# FASCICULE DE BREVET EUROPEEN

(11) **EP 0 749 489 B1**
(45) Date de publication et mention de la délivrance du brevet: **16.12.1998**
(21) Numéro de dépôt: 95911383.8
(22) Date de dépôt: 09.03.1995
(51) Int. Cl.: C12N 15/81, C12N 1/19

(54) **Cassette d'expression comportant une région promotrice issue du gène pho4 de Schizosaccharomyces pombe**
Expressionskassette, die einen aus dem Schizosaccharomyces pombe-Pho4-Gene stammenden Promotor enthält
Expression cassette comprising a promoter derived from gene pho4 of Schizosaccharomyces pombe

(30) Priorité: 10.03.1994 FR 9402767
(43) Date de publication de la demande: 27.12.1996
(73) Titulaire: TRANSGENE S.A., 67082 Strasbourg Cédex (FR)
(72) Inventeur: JACOBS, Eric, F-67120 Dorlisheim (FR); SILVESTRE, Nathalie, F-67100 Strasbourg (FR); SCHWEINGRUBER, Ernst, CH-3006 Bern (CH)
(74) Mandataire: Warcoin, Jacques
(86) Numéro de dépôt international: FR9500269
(87) Numéro de publication internationale: WO9524491

(56) Documents cités:
- DATABASE EMBL X77512, 7 Février 1994 H.FRANKHAUSER AND M.E.SCHWEINGRUBER 'Thiamine repressible genes regulatory protein THI1 (Transcription factor NTF1)' & JOURNAL OF BIOLOGICAL CHEMISTRY, vol. 269, 1994 MD US, pages 11921-11926,
- J. BIOL. CHEM. (1994), 269(16), 11921-6 CODEN: JBCHA3;ISSN: 0021-9258, Avril 1994 TANG, CAROL S. L. ET AL 'ntf1+ encodes a 6-cysteine zinc finger-containing transcription factor that regulates the nmt1 promoter in fission yeast'
- DATABASE WPI Section Ch, Week 9340 Derwent Publications Ltd., London, GB; Class B04, AN 93-314182 & IT-B-1 236 844 ( ITAL MIN UNIV RICERCA SCI TECNOLOGICA) , 22 Avril 1993
- FEBS LETT. (1992), 305(3), 244-8 CODEN: FEBLAL;ISSN: 0014-5793, NOSAKA, K. ET AL 'Upstream activation element of the PH03 gene encoding thiamin - repressible acid phosphatase in Saccharomyces cerevisiae'
- GENETICS (1992), 130(3), 445-9 CODEN: GENTAE;ISSN: 0016-6731, SCHWEINGRUBER, ANNE MARIE ET AL 'Isolation and characterization of regulatory mutants from Schizosaccharomyces pombe involved in thiamine -regulated gene expression'
- GENE (1993), 123(1), 127-30 CODEN: GENED6;ISSN: 0378-1119, MAUNDRELL, KINSEY 'Thiamin - repressible expression vectors pREP and pRIP for fission yeast'
- FEBS LETTERS, vol. 297, no. 1,2, Février 1992 AMSTERDAM NL, pages 155-158, HIROSHI NISHIMURA ET AL. 'Cloning and characteristics of a positive regulatory gene,THI2 (PHO6), of thiamin biosynthesis in Saccharomyces cerevisiae'
- J. BACTERIOL. (1994), 176(21), 6631-5 CODEN: JOBAAY;ISSN: 0021-9193, ZURLINDEN, ANDREAS ET AL 'Cloning, nucleotide sequence, and regulation of Schizosaccharomyces pombe thi4, a thiamine biosynthetic gene'
- GENE (1994), 147(1), 141-4 CODEN: GENED6;ISSN: 0378-1119, FANKHAUSER, HANS ET AL 'Thiamine -repressible genes in Schizosaccharomyces pombe are regulated by a Cys6 zinc-finger motif-containing protein'

### LISTE DE SEQUENCES

(1) INFORMATION GENERALE:
   (i) DEPOSANT:
      (A) NOM: Transgene S.A.
      (B) RUE: 11 rue de Molsheim
      (C) VILLE: Strasbourg
      (D) PROVINCE: France
      (E) PAYS: France
      (F) CODE POSTAL: 67082
      (G) TELEPHONE: (33) 88 27 91 00
      (H) TELECOPIE: (33) 88 22 58 07
   (ii) TITRE DE L' INVENTION: Usage combiné de deux cassettes d'expression pour la production d'une protéine d'intérêt
   (iii) NOMBRE DE SEQUENCES: 22
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 775 acides aminés
      (B) TYPE: acide aminé
      (D) CONFIGURATION: linéaire
   (ii) TYPE DE MOLECULE: protéine
   (iii) HYPOTHETIQUE: NON
   (iii) ANTI-SENS: NON
   (vi) ORIGINE:
      (A) ORGANISME: Schizosaccharomyces pombe
   (xi) DESCRIPTION DE LA SEQUENCE: SEQ ID NO: 1:
   Met Asn Glu Glu Ile Gly Phe Leu Lys Asn Gln Leu Phe Ala Asp Val
   1               5                   10                  15
   Lys Asp Leu Glu Arg Lys Lys Lys Arg Arg Val Pro Pro Glu Gln Arg
               20                  25                  30
   Arg Arg Val Phe Arg Ala Cys Lys His Cys Arg Gln Lys Lys Ile Lys
           35                  40                  45
   Cys Asn Gly Gly Gln Pro Cys Ile Ser Cys Lys Thr Leu Asn Ile Glu
       50                  55                  60
   Cys Val Tyr Ala Gln Lys Ser Gln Asn Lys Thr Leu Ser Arg Glu Tyr
   65                  70                  75                  80
   Leu Glu Glu Leu Ser Glu Arg Gln Leu Cys Leu Glu Tyr Ile Phe Ser
                   85                  90                  95
   Arg Met Cys Pro Asn Phe Asn Leu Glu Thr Lys Asn Leu Ile Ser Ile
               100                 105                 110
   Ser Lys Lys Leu Ser Glu Asn Glu Asn Leu Pro Val Ser Lys Ile Ala
           115                 120                 125
   Glu Val Thr Asn Glu Leu Asp Thr Leu Val Arg Ile Asn Asp Gln Leu
       130                 135                 140
   Ser Arg Asn His Ile Ser Gly Thr Thr Glu Glu Met Gln Ser Ser Ser
   145                 150                 155                 160
   Ser Leu Ile Ala Gly Glu Val Gln Pro Gly Ile Ser Phe Arg Asp Gln
                   165                 170                 175
   Leu Lys Val Gly Lys Leu Glu Asp Thr Leu Tyr Leu Gly Pro Thr Thr
               180                 185                 190
   Ser Glu Ala Phe Ile Glu Arg Leu Gln Asn Glu Leu Glu Leu Glu Ser
           195                 200                 205
   Ile Ser Glu Asp Asp Leu Tyr Ser Lys Arg Leu Ser Pro Ser Val Ser
       210                 215                 220
   Tyr Ser Glu Phe Asp Glu Gln Leu Leu Leu His Ala Arg Ser Leu Ile
   225                 230                 235                 240
   Pro Ser Lys Ala Val Val Glu Phe Leu Ile Asn Ser Phe Phe Ile Asn
                   245                 250                 255
   Val Gln Thr Asn Leu Phe Val Tyr His Pro His Phe Phe Lys Cys Arg
               260                 265                 270
   Leu Glu Ile Phe Leu Ala Met Glu Asn Gln Ile Asp Ala Gly Phe Leu
           275                 280                 285
   Cys Ile Leu Leu Met Val Leu Ala Phe Gly Asn Gln Tyr Thr Ala Glu
       290                 295                 300
   Gln Gln Glu Asp Val Ser Lys Ser Asn Phe His Ala Ser Asn Ile Gly
   305                 310                 315                 320
   Asn Arg Leu Phe Ser Ala Ala Leu Ser Ile Phe Pro Leu Val Leu Leu
                   325                 330                 335
   Gln Ser Asp Val Ser Ala Val Gln Ser Ser Leu Leu Ile Gly Leu Tyr
               340                 345                 350
   Leu Gln Ser Thr Ile Tyr Glu Lys Ser Ser Phe Ala Tyr Phe Gly Leu
           355                 360                 365
   Ala Ile Lys Phe Ala Val Ala Leu Gly Leu His Lys Asn Ser Asp Asp
       370                 375                 380
   Pro Ser Leu Thr Gln Asn Ser Lys Glu Leu Arg Asn Arg Leu Leu Trp
   385                 390                 395                 400
   Ser Val Phe Cys Ile Asp Arg Phe Val Ser Met Thr Thr Gly Arg Arg
                   405                 410                 415
   Pro Ser Ile Pro Leu Glu Cys Ile Ser Ile Pro Tyr Pro Val Ile Leu
   420 425 430
   Pro Asp Leu Glu Ile Pro Gly Ser Gln Ser Ile Val Glu Asn Met Arg
   435 440 445
   Ala Val Ile Asn Leu Ala Lys Leu Thr Asn Glu Ile Cys Asp Ser Leu
   450 455 460
   Tyr Trp Asn Pro Ser Pro Ser Phe Glu Ser Gln Val Asn Ser Val Arg
   465 470 475 480
   Arg Ile Tyr Ala Arg Leu Glu Leu Trp Lys Ser Asp Leu His Ser Ser
   485 490 495
   Val Val Phe Asp Glu Ser Ala Val Gln His Pro Leu Phe Arg Ser Asn
   500 505 510
   Ala His Val Gln Met Ile Tyr Asp Asn Ala Ile Met Leu Thr Thr Arg
   515 520 525
   Val Ile Met Val Lys Lys Leu Lys Asp Lys Asp Leu Thr Ala Glu Asn
   530 535 540
   Arg Arg Tyr Ile Gln Leu Cys Val Glu Ser Ala Thr Arg Val Ile Asn
   545 550 555 560
   Ile Ala His Leu Leu Leu Thr His Lys Cys Leu Ser Ser Leu Ser Phe
   565 570 575
   Phe Gly Leu His Val Pro Phe Ala Ser Ala Pro Ile Leu Leu Leu Ser
   580 585 590
   Leu His Tyr Glu Asn Ser Gln Asp Ile Gln Ala Val Val Thr Lys Leu
   595 600 605
   Trp Gln Val Leu Glu Phe Leu Ser Ser Arg Cys Glu Phe Ala Arg Glu
   610 615 620
   Ser Leu Asn Tyr Leu Lys Ser Phe Asn Lys Gln Leu Ser Arg Arg Asn
   625 630 635 640
   Ala Pro Asp Ile Asn Asn Pro Ile Ala Asp Phe Gln Asn Ser Phe Gln
   645 650 655
   Asn Trp Gln Ser Trp Val Gly Asp Met Ser His Gly Asp Met Leu Ser
   660 665 670
   Thr Phe Lys Leu Thr Gly Glu Ser Ser Asn Gly Ser Asn Ser Thr Pro
   675 680 685
   Asn Glu Ala Phe Gln Pro Phe Asp Gln Thr Ser Ser Leu Tyr Asn Val
   690 695 700
   Pro Gly Leu Asn Lys Ser Tyr Val Ser Asn Gln Pro Ser Leu Leu Thr
   705 710 715 720
   Pro Glu Thr Phe Leu Pro Asp Pro Val Leu Asn Leu Glu Val Asp Lys
   725 730 735
   Gln Trp Thr Ala Pro Thr Phe Leu Ser Trp Thr Glu Leu Leu Gly Pro
   740 745 750
   Thr Asn Val Ser Glu Gln Ser Ser His Thr Ala Glu Gln Thr Ser Asn
   755 760 765
   Leu Thr Leu Glu Lys Asn Gly
   770 775
(2) INFORMATION POUR LA SEQ ID NO: 2:
   (i) CARACTERISTIQUES DE LA SEQUENCE:
      (A) LONGUEUR: 642 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: gene pho4 de Schizosaccharomyces pombe
   (xi) DESCRIPTION DE LA SEQUENCE: SEQ ID NO: 2:
   CTAATAAATT AAATTGTTGG ATCTTACTAA AGACTAAATA TTAATAATAA TTTTCTCCCA    60
   AGCTATTTAT ATACATATTA GAAGCAATTT GCAAAGATAG CAGACTAATA CTCTTCAATG   120
   CCCAACTTCT TATTAGTGAT ATACATGTAA AAATATCTTT ATTATGCAAT ATTATGTAGC   180
   AGCTCGTACA ATGTTTGCAC ATCTTTACAC AATAGTATTC ATTTTGTATT ACATTATCAT   240
   TTATAATTCC ATTTCACAGA GAGTAGGCAT TGCTATTATA TAATATAAAT TTATATAAAA   300
   CAAAAAGACT GCAAAATCAT TTCCAACTGA AACTTCGTTC TTTAGTTCTA TTAAATTATT   360
   AAGTATTGGA ATATCAGATT TTTGTAAGTT CAGTAATAAT AATATCATTA TATTACACCG   420
   TTGTATTATT AATCGCATAA TATTACTGTA ACACTTTGTC CGTAATTTGC ATCGTTATTT   480
   CAGTTAACAA TTGTGGGTCC AAAATCTTAA AGTCTAATAG CGAACTACAC CAGGTTTGCA   540
   ACTTCATTCA TTTTTTTTAA TTCCAATGTA GTCGTGCCGA AATCAGACTT TGGTTTGGTG   600
   GTAGCCGGGG TGCTTAGTGT TAGCAGATAT CCGGATTGAT TG                       642
(2) INFORMATION POUR LA SEQ ID NO: 3:
   (i) CARACTERISTIQUES DE LA SEQUENCE:
      (A) LONGUEUR: 79 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique OTG2781
   (xi) DESCRIPTION DE LA SEQUENCE: SEQ ID NO: 3:
   AATTCCATTG TCTTGACTAT CACAAACTTT TAAGTCTTTT CTTTTTTGGA TCCACACCAT        60
   GGAGCTCCCG GGAGATCTA                                                     79
(2) INFORMATION POUR LA SEQ ID NO: 4:
   (i) CARACTERISTIQUES DE LA SEQUENCE:
      (A) LONGUEUR: 79 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide synthetique (OTG2782)
   (xi) DESCRIPTION DE LA SEQUENCE: SEQ ID NO: 4:
   AGCTTAGATC TCCCGGGAGC TCCATGGTGT GGATCCAAAA AAGAAAAGAC TTAAAAGTTT        60
   GTGATAGTCA AGACAATGG                                                     79
(2) INFORMATION POUR LA SEQ ID NO: 5:
   (i) CARACTERISTIQUES DE LA SEQUENCE:
      (A) LONGUEUR: 74 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG2872)
   (xi) DESCRIPTION DE LA SEQUENCE: SEQ ID NO: 5:
   GATCCAACCA CATAATGTTC TTGCAAAATT TATTCCTTGG CTTTTTGGCC GTCGTTTGTG 60
   CCAACGCGTG AGCT 74
(2) INFORMATION POUR LA SEQ ID NO: 6:
   (i) CARACTERISTIQUES DE LA SEQUENCE:
      (A) LONGUEUR: 66 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide synthetique (OTG2873)
   (xi) DESCRIPTION DE LA SEQUENCE: SEQ ID NO: 6:
   CACGCGTTGG CACAAACGAC GGCCAAAAAG CCAAGGAATA AATTTTGCAA GAACATTATG 60
   TGGTTG 66
(2) INFORMATION POUR LA SEQ ID NO: 7:
   (i) CARACTERISTIQUES DE LA SEQUENCE:
      (A) LONGUEUR: 11 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG2874)
   (xi) DESCRIPTION DE LA SEQUENCE: SEQ ID NO: 7:
   CGCGATTACG T 11
(2) INFORMATION POUR LA SEQ ID NO: 8:
   (i) CARACTERISTIQUES DE LA SEQUENCE:
      (A) LONGUEUR: 9 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide synthetique (OTG2875)
   (xi) DESCRIPTION DE LA SEQUENCE: SEQ ID NO: 8:
   ATACGTAAT 9
(2) INFORMATION POUR LA'SEQ ID NO: 9:
   (i) CARACTERISTIQUES DE LA SEQUENCE:
      (A) LONGUEUR: 43 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG3569)
   (xi) DESCRIPTION DE LA SEQUENCE: SEQ ID NO: 9:
(2) INFORMATION POUR LA SEQ ID NO: 10:
   (i) CARACTERISTIQUES DE LA SEQUENCE:
      (A) LONGUEUR: 43 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide synthetique (OTG3239)
   (xi) DESCRIPTION DE LA SEQUENCE: SEQ ID NO: 10:
   GAGAAATACC ACTTAACTTC ATGGATCCCG AGAAAAAACA ATG 43
(2) INFORMATION POUR LA SEQ ID NO: 11:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide synthetique (OTG3210)
   (xi) DESCRIPTION DE LA SEQUENCE: SEQ ID NO: 11:
   CCCAAACCAT GGCAATCAAT CCGGATATCT GCTAAC 36
(2) INFORMATION POUR LA SEQ ID NO: 12:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG4645)
   (xi) DESCRIPTION DE LA SEQUENCE: SEQ ID NO: 12:
   AAACAAGCAT GCGTTTGGTG GTAGCCGGGG 30
(2) INFORMATION POUR LA SEQ ID NO: 13:
   (i) CARACTERISTIQUES DE LA SEQUENCE:
      (A) LONGUEUR: 40 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG4646)
   (xi) DESCRIPTION DE LA SEQUENCE: SEQ ID NO: 13:
   AAACAAGCAT GCTCATTCAT TTTTTTTAAT TCCAATGTAG 40
(2) INFORMATION POUR LA SEQ ID NO: 14:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG4647)
   (xi) DESCRIPTION DE LA SEQUENCE: SEQ ID NO: 14:
   AAACAAGCAT GCGGTCCAAA TCTTAAAGTC TAATAGCG 38
(2) INFORMATION POUR LA SEQ ID NO: 15:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG4648)
   (xi) DESCRIPTION DE LA SEQUENCE: SEQ ID NO: 15:
   AAACAAGCAT GCTACTGTAA CACTTTGTCC GTAATTTGC 39
(2) INFORMATION POUR LA SEQ ID NO: 16:
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG4649)
   (xi) DESCRIPTION DE LA SEQUENCE: SEQ ID NO: 16:
   AAACAAGCAT GCCACAGAGA GTAGGCATTG CTATT 35
(2) INFORMATION POUR LA SEQ ID NO: 17:
   (i) CARACTERISTIQUES DE LA SEQUENCE:
      (A) LONGUEUR: 42 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG4924)
   (xi) DESCRIPTION DE LA SEQUENCE: SEQ ID NO: 17:
   CAGCCGGGGT GCTTAGTGTT AGCAGATATC CGGATTGATT GC 42
(2) INFORMATION POUR LA SEQ ID NO: 18:
   (i) CARACTERISTIQUES DE LA SEQUENCE:
      (A) LONGUEUR: 50 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide synthetique (OTG4925)
   (xi) DESCRIPTION DE LA SEQUENCE: SEQ ID NO: 18:
   CATGGCAATC AATCCGGATA TCTGCTAACA CTAAGCACCC CGGCTGCATG 50
(2) INFORMATION POUR LA SEQ ID NO: 19:
   (i) CARACTERISTIQUES DE LA SEQUENCE:
      (A) LONGUEUR: 72 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG5296)
   (xi) DESCRIPTION DE LA SEQUENCE: SEQ ID NO: 19:
   CGGCCCATGG TTTGGTGGTA GCCGGGGTGC TTAGTGTTAG CAGATATCCG GATTGATTGC 60
   CATGGGCCTC TC 72
(2) INFORMATION POUR LA SEQ ID NO: 20:
   (i) CARACTERISTIQUES DE LA SEQUENCE:
      (A) LONGUEUR: 72 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide synthetique (OTG5297)
   (xi) DESCRIPTION DE LA SEQUENCE: SEQ ID NO: 20:
   AGGCCCATGG CAATCAATCC GGATATCTGC TAACACTAAG CACCCCGGCT ACCACCAAAC 60
   CATGGGCCGG AG 72
(2) INFORMATION POUR LA SEQ ID NO: 21:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide synthetique (OTG5217)
   (xi) DESCRIPTION DE LA SEQUENCE: SEQ ID NO: 21:
   CCCAAAGCAT GCAAGCTTAA AGGAATCCGA TTGTCATTC 39
(2) INFORMATION POUR LA SEQ ID NO: 22:
   (i) CARACTERISTIQUES DE LA SEQUENCE:
      (A) LONGUEUR: 37 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide synthetique (OTG3028)
   (xi) DESCRIPTION DE LA SEQUENCE: SEQ ID NO: 22:
   ATCTTGTTAG TAGCCATGGA TCCGATTTAA CAAAGCG                                37

## Description

La présente invention se rapporte au domaine des biotechnologies, notamment à un perfectionnement apporté à la production d'une protéine hétérologue d'intérêt commercial ou thérapeutique dans une cellule eucaryote et, en particulier, une levure du genre *Schizosaccharomyces.* Elle concerne une cassette d'expression dans laquelle le fragment d'ADN codant pour la protéine d'intérêt est placé sous le contrôle d'une région promotrice isolée ou dérivée du gène *pho4* de *Schizosacccharomyces pombe.*

Depuis quelques années déjà, de nombreuses cassettes d'expression pour la production de protéines d'intérêt dans les cellules eucaryotes ont été décrites dans la littérature. Ces cassettes comprennent notamment une région promotrice fonctionnelle dans la cellule considérée. D'une manière générale, une région promotrice est située dans la région 5' flanquante des gènes et comprend l'ensemble des éléments permettant la transcription d'un fragment d'ADN placé sous leur dépendance. Une région promotrice peut également être constituée par l'assemblage d'éléments de diverses origines, fonctionnels dans la cellule hôte, comme notamment :
- une région promotrice minimale comprenant la TATA box et le site d'initiation de la transcription. Si elle semble nécessaire à une initiation correcte de la transcription, elle ne peut, à elle seule, assurer une transcription efficace ; et
- des régions situées en amont de la TATA box qui permettent d'assurer un niveau efficace de transcription soit de manière constitutive (niveau de transcription constant tout au long du cycle cellulaire, quelles que soient les conditions de culture) soit de manière régulable (dites régions de régulation permettant une activation de la transcription en présence d'un activateur et/ou répression de la transcription en présence d'un répresseur), suivant le gène dont elles sont issues.

Lorsqu'on cherche à obtenir de grande quantité d'une protéine d'intérêt, on utilise généralement une région promotrice forte et constitutive pour contrôler l'expression du fragment d'ADN codant pour la protéine d'intérêt, par exemple les régions 5' flanquantes des gènes PGK (3-phosphoglycérate kinase) de *Saccharomyces cerevisiae* (Hitzeman et al., 1983, Science, 219, 620-625) et *adh* (alcool déshydrogénase) de *Schizosaccharomyces pombe* (Russel et Hall, 1983, J. Biol. Chem., 258, 143-149).

Cependant, l'utilisation d'une région promotrice forte et constitutive n'est pas adaptée à la production de protéines d'intérêt présentant une certaine toxicité vis à vis de la cellule hôte . En effet, la production de protéines toxiques affecte la croissance des cellules et risque d'entraîner la sélection de mutations spontanées conduisant à une perte ou une réduction notable du taux de production. Dans les cas extrêmes, la viabilité des cellules peut être affectée.

Pour ces raisons, il peut être avantageux de disposer de régions promotrices régulables permettant de faire varier la production de la protéine d'intérêt en fonction des conditions de culture ou de la phase de croissance cellulaire. D'une manière générale, de telles régions promotrices sont isolées ou dérivent de gènes régulables.

Au cours de ces dernières années, un certain nombre de gènes régulables ont été mis en évidence dans de nombreux eucaryotes et notamment dans *Schizosaccharomyces pombe.* Des mécanismes très variés gouvernent la régulation de ces gènes. A titre d'exemples de gènes régulables de *Schizosaccharomyces pombe,* on peut citer les gènes de "heat shock" dont l'expression augmente avec la température (Gallo et al., 1991, Mol. Cell. Biol., *11*, 281-288) et le gène codant pour l'enzyme fructose biphosphatase (*fbp*) pour lequel la transcription est réprimée en présence de glucose et induite en condition de carence en glucose (Hoffman et Winston, 1989, Gene, 84, 473-479). Entrent également dans cette catégorie les gènes régulables par la thiamine, à savoir les gènes *pho4* (Yang et Schweingruber, 1990, Curr. Genet., 18, 269-272), *nmtl* (Maundrell, 1990, J. Biol. Chem., *265,* 10857-10864) et *thi2* (Zurlinden and Schweingruber, 1992, Gene, 117, 141-143) dont l'expression est régulée au niveau transcriptionnel par la thiamine, plus précisément réprimée en présence de thiamine et induite ou déréprimée en son absence.

On a maintenant caractérisé les régions de régulation de la région promotrice du gène *pho4* impliquées dans la réponse à la thiamine et généré des cassettes d'expression régulables pour la production de protéines d'intérêt dans *Schizosaccharomyces.* Les levures hébergeant une telle cassette d'expression sont cultivées dans un milieu supplémenté en thiamine lorsque la culture vise uniquement à leur propagation. Dès lors qu'une culture est entreprise en vue de produire une protéine d'intérêt, les levures sont transférées dans un milieu dépourvu de thiamine. Ainsi, lorsque ces régions sont placées en amont d'un fragment d'ADN codant pour le variant Lys 47 de l'hirudine, on obtient une expression efficace en l'absence de thiamine, au moins équivalente à celle détectée avec la région promotrice forte du gène *adh.* De plus, leur mise en oeuvre dans une cassette d'expression de la protéine CFTR (Cystic Fibrosis Transmembrane Regulator) a permis sa production par voie recombinante, pourtant toxique dans *Schyzosaccharomyces pombe,*

On a également trouvé un gène de *Schizosaccharomyces pombe* codant pour un produit activateur agissant sur l'expression des gènes régulables par la thiamine dans des conditions inductrices (en absence de thiamine). L'amplification de ce gêne, notamment par introduction dans un vecteur multicopie et transformation d'une souche de *Schizosaccharomyces pombe,* laquelle comprend également un grand nombre de copies d'un fragment d'ADN codant pour une protéine d'intérêt, placé sous la dépendance d'une région promotrice régulable par la thiamine, pourrait permettre d'améliorer les niveaux de la production de la protéine d'intérêt en fonction des conditions de culture, en particulier en absence de thiamine.

Traditionnellement, la thiamine est ajoutée dans les milieux de culture des levures. Le fait de ne pas en mettre représente un coût moindre et n'affecte pas ou peu leur viabilité. Ces différents critères, qui satisfont aux conditions de production à l'échelle industrielle, illustrent les avantages de la présente invention.

Par conséquent, la présente invention a pour objet
une cassette d'expression comportant un fragment d'ADN codant pour une protéine d'intérêt, placé sous le contrôle des éléments nécessaires à son expression ; lesdits éléments comprenant notamment une région promotrice régulable par la thiamine et issue du gène *pho4*, de *Schizosaccharomyces pombe*.

Aux fins de la présente invention, le fragment d'ADN peut être issu d'un organisme eucaryote, procaryote ou d'un virus. Il peut être isolé par toute technique en usage dans le domaine de l'art, par exemple par clonage, PCR (Polymerase Chain Reaction) ou synthèse chimique. D'autre part il peut coder pour une protéine d'intérêt (i) intracellulaire (ii) membranaire présente à la surface de la levure hôte ou (iii) sécrétée dans le milieu de culture. Il peut donc comprendre des éléments additionnels appropriés comme, par exemple, une séquence codant pour un signal de sécrétion. De tels éléments sont connus de l'homme de l'art.

Par ailleurs, le fragment d'ADN peut coder pour une protéine d'intérêt correspondant à tout ou partie d'une protéine native, telle que trouvée dans la nature. Il peut également s'agir d'une protéine chimérique, par exemple provenant de la fusion de polypeptides d'origines diverses, ou d'un mutant présentant des propriétés biologiques améliorées et/ou modifiées. Un tel mutant peut être obtenu par les techniques de biologie moléculaire. Parmi les protéines d'intérêt au sens de la présente invention, on peut citer plus particulièrement :
- les cytokines et notamment les interleukines, les interférons, les facteurs de stimulation des colonies (CSF) et les facteurs de croissance ;
- les anticoagulants, de préférence l'hirudine, notamment les variants de l'hirudine décrits dans la demande européenne EP 332 523 et tout particulièrement le variant HV2 Lys 47 ;
- les enzymes telles que la trypsine et les ribonucléases ;
- les inhibiteurs d'enzymes tels que l'α l-antitrypsine, l'antithrombine III et les inhibiteurs de protéases virales ;
- les protéines impliquées dans les canaux ioniques, telles que la protéine CFTR dont la séquence est décrite dans Riordan et al. (1989, Science, *245*, 1066-1073) ;
- les protéines capables d'inhiber l'initiation ou la progression de cancers, telles que les produits d'expression des gènes suppresseurs de tumeurs, par exemple les gènes p53 et Rb ; et
- les protéines capables d'inhiber une infection virale ou son développement, par exemple les épitopes antigéniques de ces virus ou des variants altérés de protéines virales susceptibles de compétition avec les protéines virales natives.

Bien entendu, ces exemples ne sont pas limitatifs. D'une façon générale, on aura recours pour l'expression du fragment d'ADN à une région promotrice fonctionnelle dans la cellule considérée ci régulable par la thiamine. Cette dernière est isolée de la région 5' flanquante des gênes régulables par la thiamine, tels que ceux mentionnés précédemment. Bien entendu, elle peut être modifiée par mutation, délétion et/ou addition d'un ou plusieurs nucléotides(s) par rapport à la région promotrice native, à la condition que ces modifications n'altèrent pas de manière drastique sa capacité de régulation. D'une façon générale, tout ou partie d'une telle région promotrice peut être utilisée dans le cadre de la présente invention. Ainsi, on peut mettre en oeuvre une région promotrice d'un gêne régulable par la thiamine, comprenant une région de régulation capable de conférer la régulation par la thiamine et une TATA box homologue audit gêne régulable.

Selon un autre mode de réalisation, on peut employer une région de régulation issue d'un gêne régulable par la thiamine placée en amont d'une région promotrice minimale comprenant une TATA box d'une origine quelconque, capable d'assurer une initiation correcte de la transcription d'un premier fragment d'ADN dans la cellule considérée. L'homme du métier connaît de telles régions promotrices minimales. On peut citer à titre d'exemples celles des gênes *adh* de *Schizosaccharomyces pombe* et TEI de CMV (Cytomegalovirus) (Boshan et al., 1985, Cell, *41*, 521-530).

"Région de régulation" se réfère à une séquence nucléotidique de taille variable, capable de conférer la régulation par la thiamine, c'est à dire d'induire en absence de thiamine une expression du fragment d'ADN placé sous sa dépendance à un niveau notablement plus élevé qu'en présence de thiamine. Une région de régulation comprend notamment un ou des éléments d'activation et/ou de répression responsables de la régulation.

Bien qu'une seule région de régulation soit suffisante pour assurer une régulation par la thiamine, on peut également envisager d'employer plusieurs régions de régulation en tandem pour augmenter les niveaux d'expression. Selon un usage selon la présente invention, on peut mettre en oeuvre notamment de 1 à 25 régions de régulation, avantageusement de 1 à 7 et, de préférence, de 1 à 4. Par ailleurs, la ou les région(s) de régulation peut (peuvent) être insérée(s) en amont d'une région promotrice minimale dans une orientation sens ou inversée par rapport à la TATA box. De préférence, cette région de régulation est placée immédiatement en amont de la TATA box c'est à dire à une distance de 1 à 35pb, avantageusement de 1 à 20pb, de préférence de 1 à 10pb et de, manière tout à fait préférée, de 1 à 6pb.

Dans le cadre de la présente invention, on aura, comme vu plus haut, recours à une région promotrice issue du gène *pho4* de *Schizosaccharomyces pombe.* Dans ce contexte, les inventeurs ont caractérisé une région de régulation de 40pb localisée juste en amont de la TATA box. Ainsi, selon un mode avantageux, une région de régulation en usage dans la présente invention comprend au moins 17 nucléotides de la séquence telle que montrée dans l'identificateur de séquence NO:2 et débutant au nucléotide en position +603 et se terminant au nucléotide en position +642. La présente invention inclut également toute séquence capable de s'hybrider dans des conditions stringentes avec une telle séquence ainsi que son complémentaire. Bien entendu, une région de régulation au sens de l'invention peut être plus grande et comprendre plus de séquence de la région promotrice du gène *pho4*,

A titre d'exemples non limitatifs, on peut envisager d'employer une région de régulation ayant une séquence telle que montrée dans l'identificateur de séquence NO: 2,
- débutant au nucléotide en position +603 et se terminant au nucléotide en position +642;
- débutant au nucléotide en position +593 et se terminant au nucléotide en position +642;
- débutant au nucléotide en position +544 et se terminant au nucléotide en position +642;
- débutant au nucléotide en position +496 et se terminant au nucléotide en position +642;
- débutant au nucléotide en position +444 et se terminant au nucléotide en position +642;
- débutant au nucléotide en position +255 et se terminant au nucléotide en position +642; ou
- débutant au nucléotide en position +1 et se terminant au nucléotide en position +642.

Une construction particulièrement intéressante est celle qui associe une région de régulation issue du gène *pho4* de *Schizosaccharomyces pombe* et une région promotrice minimale issue du gène *adh* de *Schizosaccharomyces pombe.* Cette dernière correspond à la séquence telle que publiée dans Russel et al. (1983, *supra)* et comprise entre les nucléotides -119 à -12. Il va de soit qu'elle peut comporter des modifications (mutation, délétion et/ou addition d'un ou plusieurs nucléotides) par rapport à la séquence publiée du moment que ces modifications ne réduisent pas d'une manière drastique sa capacité à initier la transcription.

D'autre part, la cassette d'expression de la présente invention peut, en outre, contenir d'autres éléments contribuant à l'expression du fragment d'ADN, notamment une séquence de terminaison de la transcription telle que celle du gène *arg 3* de *Schizosaccharomyces pombe* (Van Huffel et al., 1992, Eur. J. Biochem., *205,* 33-43) ainsi qu'un enhancer de transcription fonctionnel dans la cellule considérée, par exemple, l'enhancer du gène IEl du CMV.

Une deuxième cassette d'expression peut coder pour un produit activateur capable d'activer l'expression du fragment d'ADN placé sous le contrôle d'une région promotrice régulable par la thiamine comme ci-dessus décrit et appelé dans ce cas, ci-après : "premier fragment d'ADN". Le terme "produit activateur" désigne un polypeptide capable d'interagir soit directement avec la région de régulation impliquée dans la régulation par la thiamine soit indirectement par l'intermédiaire de facteurs cellulaires. Il s'agit de préférence d'un produit activateur agissant au niveau transcriptionnel. Différents gènes ou parties de gènes isolés de cellules eucaryotes variées et codant pour un produit activateur de l'expression des gènes régulables par la thiamine peuvent être utilisés dans le cadre de la présente invention. De tels gènes activateurs peuvent être obtenus par toute technique conventionnelle dans le domaine de l'art et, notamment, selon la technique décrite dans l'exemple 2 par complémentation d'une cellule mutante présentant une absence de dérépression de l'expression des gènes normalement induits en absence de thiamine.

Mais on préfère tout particulièrement mettre en oeuvre un gène issu de *Schizosaccharomyces pombe* codant pour un produit activateur ayant la séquence telle que montrée dans l'identificateur de séquence NO:1 débutant à l'acide aminé en position +1 et se terminant à l'acide aminé en position +775 ou un variant fonctionnel dudit produit activateur. Par "vanant fonctionnel", on entend un polypeptide capable d'exercer une fonction activatrice de l'expression des gènes régulables par la thiamine. De tels variants fonctionnels peuvent être obtenus par mutation, délétion, substitution et/ou addition d'un ou plusieurs résidus acide aminé. Ces modifications peuvent être réalisées selon les techniques classiques de biologie moléculaire. La fonctionnalité du variant ainsi obtenu peut être confirmée selon la technique indiquée dans l'exemple 2, par transformation des fragments d'ADN codant pour chacun de ces variants dans des souches mutantes et mesure de la dérépression par restauration d'une activité enzymatique appropriée.

La deuxième cassette d'expression peut être comprise dans un vecteur à réplication autonome et, dans ce cas, le deuxième fragment d'ADN peut être sous la dépendance soit de sa propre région promotrice, soit d'une deuxième région promotrice hétérologue audit fragment d'ADN. Selon une autre variante, la deuxième cassette d'expression est insérée dans le génome de la cellule hôte, à la condition que le deuxième fragment d'ADN soit placé sous le contrôle d'une deuxième région promotrice hétérologue audit fragment d'ADN.

S'agissant d'une deuxième région promotrice hétérologue, elle peut être constitutive ou régulable, d'une origine quelconque du moment qu'elle soit fonctionnelle dans la cellule hôte. Le choix d'une telle région promotrice est à la portée de l'homme du métier. On peut, néanmoins, citer les régions promotrices des gènes *adh* et *fbp* de *Schizosaccharomyces pombe* et du gène IEI du CMV.

Cependant, il peut être avantageux d'avoir recours à une deuxième région promotrice régulable par la thiamine du type de la (première) région promotrice ci-dessus décrite.

Comme précédemment, la deuxième cassette d'expression peut contenir d'autres éléments nécessaires à l'expression du deuxième fragment d'ADN tels que ceux mentionnés ci-dessus.

Selon la variante, par ailleurs préférée, où une ou plusieurs copie(s) de la deuxième cassette d'expression est (sont) insérée(s) dans un vecteur d'expression, on emploie notamment un vecteur d'expression multicopie et, en particulier, un vecteur comprenant une ou plusieurs copie(s) de la première cassette d'expression. L'insertion de la deuxième cassette d'expression en usage dans la présente invention se fait de préférence en dehors de la première cassette d'expression proprement dite. Un tel vecteur peut comporter par ailleurs des éléments assurant sa réplication, c'est à dire une origine de réplication telle que l'origine *arsl* de *Schizosaccharomyces pombe* et, de façon optionnelle, *ori d'Escherichia coli.* En outre, il peut également comprendre des gènes de sélection, tels que le gène *URA3* ou *LEU2* de *Saccharomyces cerevisiae,* le gène *ura4* ou *LEU1*, de *Schizosaccharomyces pombe* ou un gène de résistance à un antibiotique. On préfère tout particulièrement mettre en usage un vecteur multicopie présent entre 20 et 500 copies dans la cellule hôte, avantageusement entre 25 et 400 copies, et de préférence entre 50 et 300 copies.

Dans le cadre de la présente invention, les premières et deuxièmes cassettes d'expression peuvent être présentes dans une cellule hôte selon un rapport en nombre de copies de 200:1, avantageusement de 25:1, de préférence 10:1 et de manière tout à fait préférée de 1:1.

La présente invention s'étend également à :
(i) un vecteur d'expression comprenant une troisième cassette d'expression selon l'invention ; et
(ii) une cellule hôte comprenant une troisième cassette d'expression ou un vecteur d'expression selon l'invention. On préfère tout particulièrement une cellule de levure avantageusement du genre *Schizosaccharomyces* et, de préférence, de l'espèce *pombe.*

Enfin, la présente invention vise également un procédé de production d'une protéine d'intérêt par une cellule hôte selon l'invention, notamment une cellule de *Schizosaccharomyces pombe*, selon lequel :
- on cultive dans un milieu approprié en absence de thiamine ladite cellule hôte ; et
- on récupère ladite protéine d'intérêt.

Dans le cadre de l'invention, la protéine d'intérêt peut être récupérée directement dans le milieu de culture ou après lyse des cellules selon les techniques conventionnelles. Par ailleurs, elle peut être purifiée en appliquant les techniques standards connues de l'homme de l'art, par exemple et à titre indicatif, par chromatographie ou immunopurification.

Les exemples ci-après permettront de mettre en évidence d'autres caractéristiques et avantages de la présente invention. Ces exemples sont illustrés par référence aux figures suivantes :

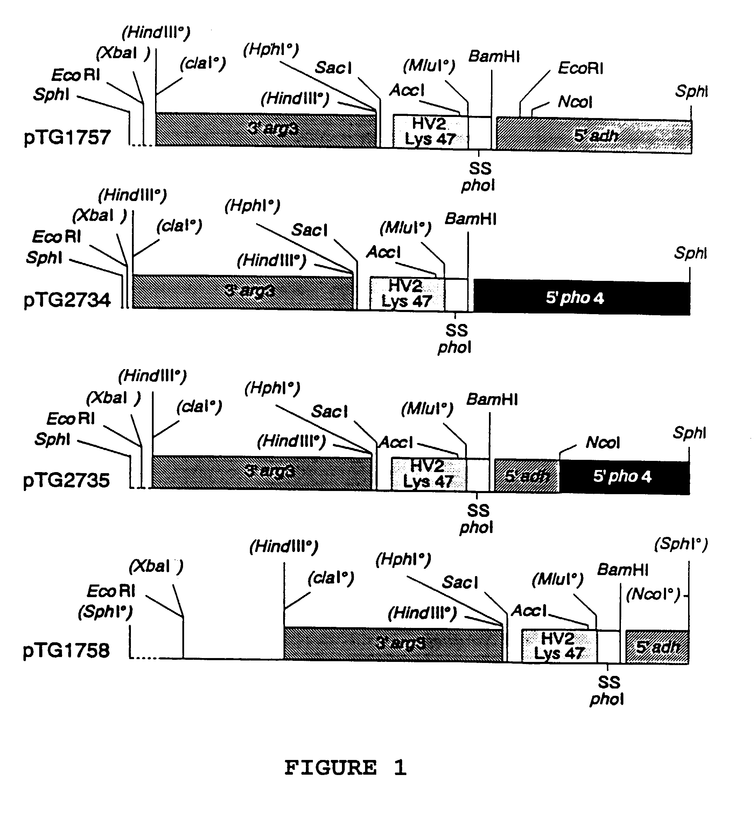

La Figure 1 est une représentation schématique des cassettes pour l'expression du fragment d'ADN codant pour HV2 Lys 47 placé sous la dépendance d'une région promotrice régulable par la thiamine (pTG2734 et pTG2735), constitutive (pTG1757) ou non-fonctionnelle (pTG1758).

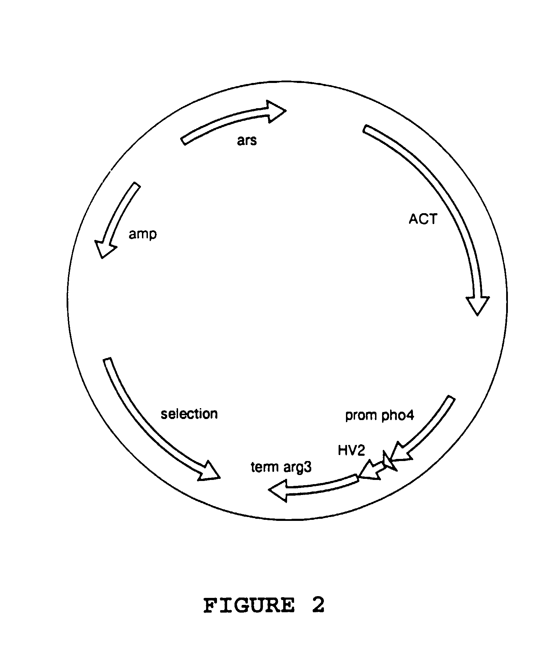

La Figure 2 est une représentation schématique d'un vecteur d'expression comportant une première cassette d'expression du variant Lys 47 de l'hirudine (HV2) sous le contrôle du promoteur *pho*4 et un gène activateur (ACT) capable d'activer le promoteur *pho4,* de même qu'un origine de réplication (ars) et des gènes codant pour des marqueurs de sélection (amp et sélection).

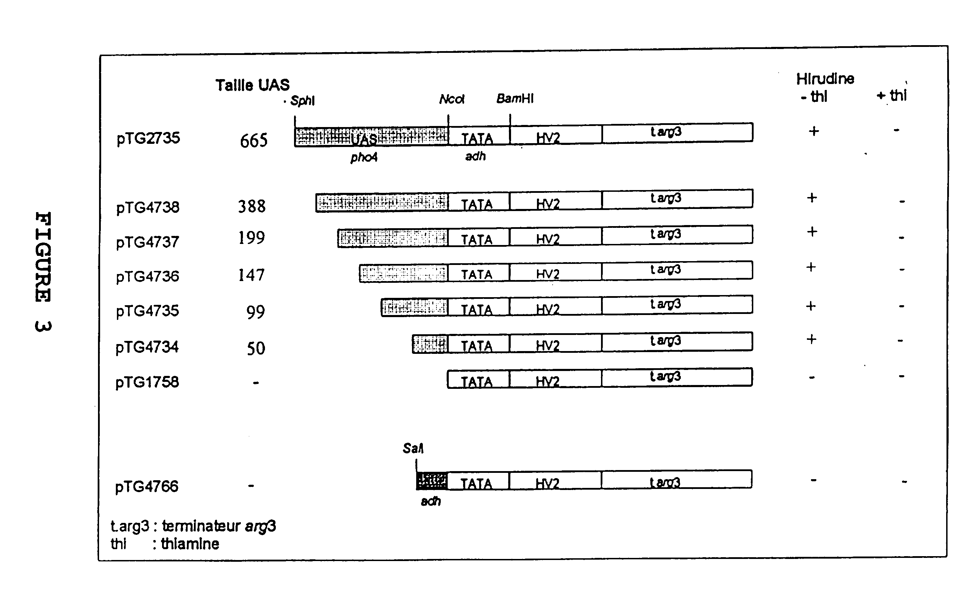

La Figure 3 est une représentation schématique des vecteurs de délétion de la région régulatrice (UAS) du gène *pho4* (pTG4734 à pTG4738) et des vecteurs témoins (pTG2735 : région régulatrice *pho4* complète ; pTG1758 : région promotrice minimale *adh* et pTG4766 : région promotrice minimale *adh* complétée de 30pb de séquence 5' et d'un site *Sal*I). La capacité de ces vecteurs à produire et sécréter l'hirudine (HV2) après culture dans un milieu dépourvu ou contenant de la thiamine (-thi ou +thi) est indiqué succintement (+ ou -).

Les constructions décrites ci-après sont réalisées selon les techniques générales de génie génétique et de clonage moléculaire détaillées dans Maniatis et al. (1989, Laboratory Manual, Cold Spring Harbor Laboratory Press, Cold Spring Harbor, NY). L'ensemble des étapes de clonage mettant en oeuvre des plasmides bactériens est effectué par passage dans la souche E. coli 5K.

On utilise pour introduire les différents vecteurs dans les souches de *Schizosaccharomyces pombe* la technique à l'acétate de lithium (Ito et al., 1983, J. Bacteriol., *153,* 163-168). Mais toute autre technique standard peut être employée.

### EXEMPLE 1 : Construction de vecteurs d'expression dans lesquels le gène codant pour le variant HV2 Lys 47 est placé sous le contrôle d'une région promotrice dérivée du gène pho4 de Schizosaccharomyces pombe.

*A. Vecteur pTG2734 dans lequel le gène HV2 Lys 47 est placé sous le contrôle de la région promotrice isolée dit gène pho4 de Schizosaccharomyces pombe.*
Le plasmide pEVp11 (Russell et Nurse, 1986, Cell, 45, 145-153) contient la région promotrice du gène *adh* de *Schizosaccharomyces pombe* sous forme d'un fragment *Sph*I*-Eco*RI de 700pb, le site *Eco*RI étant situé dans la région 5' du gène *adh* 59pb en amont de l'ATG initiateur. Il est digéré par les enzymes *Eco*RI et *Hind*III et on introduit un fragment synthétique résultant de la réassociation des oligonucléotides simple brin OTG2781 et OTG2782 (décrits respectivement dans les SEQ ID NO: 3 et NO: 4) dans le but de compléter la région promotrice *adh* jusqu'à 11 pb en amont de l'ATG initiateur et de créer des sites de restriction appropriés facilitant les étapes de clonage ultérieures. On génère pTG1702 dans lequel on insère en aval de la région promotrice *adh* une séquence codant pour le signal de sécrétion de la phosphatase acide majeure de *Schizosaccharomyces pombe* (phol). Pour ce faire, un fragment d'ADN synthétique issu de la réassociation des oligonucléotides OTG2872 et OTG2873 (SEQ ID NO: 5 et NO: 6) est ligué à pTG1702 préalablement digéré par *Bam*HI et *Sac*I*.* On obtient pTG1716.
Ce dernier est modifié par l'insertion d'une séquence d'ADN codant pour le variant Lys 47 de l'hirudine (HV2 Lys47). pTG1716 est digéré par *Mlu*I et HindIII avant d'être ligué d'une part à un fragment synthétique présentant des extrémités protubérantes *Mlu*I et *Acc*I et résultant de la réassociation des oligonucléotides OTG2874 et OTG2875 (décrits respectivement dans les SEQ ID NO: 7' et NO: 8) et d'autre part à un fragment *Acc*I-*Hin*dIII isolé du vecteur pTG2974. Ce dernier porte notamment un fragment d'ADN codant pour HV2 Lys 47.
On complète la cassette d'expression par l'introduction en 3' du gène HV2 Lys 47 d'une séquence terminatrice de transcription correspondant à la région 3' du gène *arg3* de *Schizosaccharomyces pombe.* Un fragment *Hpa*I-*Cla*I de 0,92 kb est isolé de pCVH3 (Van Huffel et al., 1992, Eur J. Biochem., 205, 33-43) puis traité au fragment Klenow de l'ADN polymérase. Ce fragment comporte les derniers codons du gène *arg3* de *Schizosaccharomyces pombe* suivis de la séquence de terminaison de la transcription. Il est inséré dans pTG1702 digéré par *Hind*III et dont les extrémités ont été rendues franches par traitement au fragment Klenow de l'ADN polymérase, pour donner pTG1746.
pTG1746 est digéré par *Xba*I*,* traité au fragment Klenow de l'ADN polymérase avant d'être digéré par *BamHI.* Le fragment comportant les séquences de terminaison de la transcription est introduit dans le vecteur pDW230 digéré par *Eco*RI*,* soumis à un traitement au fragment Klenow de l'ADN polymérase puis digéré par *Bam*HI. On obtient pTG1751.
Le vecteur pDW230 est similaire à pDW232 décrit dans la littérature (Weilguny et al., 1991, Gene, 99, 47-54). Ils dérivent tous deux de pGEM3 dans lequel on a inséré une origine de réplication fonctionnelle dans *Schizosaccharomyces pombe* (origine *arsl)* ainsi que le gène *ura4* de *Schizosaccharomyces pombe* en tant que marqueur de sélection ; à la différence que l'introduction du fragment portant l'origine *arsl* au niveau du site *Nae*I de pGEM3, donnant lieu à pDW230, a entraîné une délétion de ce site jusqu'à la base 2862.
Le fragment *Sph*I*-Sac*I comportant la région promotrice *adh* suivie de la séquence codant pour le signal de sécrétion phol et HV2 Lys 47 est isolé de pTG1722. Il est ensuite sous-cloné entre les mêmes sites de pTG1751. Il en résulte pTG1757 dans lequel l'expression de HV2 Lys 47 est contrôlée par la région promotrice *adh* et est donc constitutive (Figure 1).
D'autre part, la région promotrice du gène *pho4* de *Schizosaccharomyces pombe* est obtenue par PCR (Polymérase Chain Reaction) à partir du clone pSp4B (Yang and Schweingruber, 1990, Current Genet., *18*, 269-272) et à l'aide des amorces OTG3569 comportant un site *Sph*I (SEQ ID NO: 9) et OTG3239 muni d'un site *Bam*HI (SEQ ID NO: 10).
Le fragment *Sph*I*-Bam*HI ainsi généré est substitué au fragment *Sph*I*-Bam*HI portant la région promotrice *adh* de pTG1757 pour donner pTG2734 (Figure 1).
B. *Construction de vecteurs d'expression de HV2 Lys 47 comprenant une région promotrice hybride "pho4-adh" et caractérisation des régions du gène pho4 impliquées dans la régulation par la thiamine.*
On isole par PCR et à partir du vecteur pTG2734, un fragment de 642 pb comportant les séquences 5' flanquantes du gène *pho4* de *Schizosaccharomyces pombe* situées en amont de la TATA box. On met en oeuvre les amorces OTG3569 (SEQ ID NO: 9) et OTG3210 (SEQ ID NO: 11).
Le fragment PCR *Sph*I*-Nco*I ainsi obtenu, est introduit dans pTG1757 digéré par les mêmes enzymes pour donner pTG2735 (Figure 1).
Afin de localiser les régions régulatrices du gène *pho4* responsables de la régulation par la thiamine , un certain nombre de fragments PCR ont été générés à partir de pTG2734 et de l'amorce OTG3210 précédemment décrite combinée à l'une des amorces suivantes (voir tableau 1) :

**Tableau 1**

| Amorces | Vecteur d'expression | Taille de la région 5' *pho4* synthétisée |
|---|---|---|
| OTG4645 (SEQ ID NO: 12) | pTG4734 | 50 pb |
| OTG4646 (SEQ ID NO: 13) | pTG4735 | 99 pb |
| OTG4647 (SEQ ID NO: 14) | pTG4736 | 147 pb |
| OTG4648 (SEQ ID NO: 15) | pTG4737 | 199 pb |
| OTG4649 (SEQ ID NO: 16) | pTG4738 | 388 pb |

Chacun des fragments PCR *Sph*I*-Nco*I généré est sous cloné comme précédemment dans le vecteur pTG1757 traité par les mêmes enzymes. Une représentation schématique de ceux-ci fait l'objet de la Figure 3.
Enfin, on génère le vecteur pTG5701 par introduction dans pTG1757 d'un fragment d'ADN muni d'extrémités *Sph*I et *Nco*I et provenant de la réassociation des oligonucléotides OTG4924 et OTG4925 (SEQ ID NO: 17 et 18). Ainsi dans pTG5701, l'expression de HV2 Lys 47 est placé sous le contrôle d'une région de régulation de 40 pb du gène *pho4* précédant la TATA box du gène *adh.*
*C. Construction d'un vecteur d'expression de HV2 Lys 47 comprenant une région promotrice hybride "pho4-adh" et dans laquelle la région de régulation pho4 est en orientation anti-sens par rapport à la TATA box du gène adh.*
Le vecteur pTG4734 est digéré par *Sph*I et *Nco*I puis traité à l'ADN polymérase T4 puis religué. On vérifie par séquençage selon les méthodes traditionnelles, les clones comportant une copie d'une région de régulation du gène *pho4* de 50 pb mais en orientation reverse par rapport au vecteur d'origine pTG4734 et donc à la TATA box du gène *adh.*
*D. Construction de vecteurs d'expression de HV2 Lys 47 comprenant une région promotrice hybride "pho4-adh" incluant plusieurs régions de rrégulation pho4 de 50 pb en tandem.*
Un fragment d'ADN correspondant à une région de régulation du gène *pho4* de 50 pb muni à ses deux extrémités d'un site de restriction *Bgl*I, est généré par réassociation des oligonucléotides OTG5296 et OTG5297 (SEQ ID NO: 19 et 20). L'étape de réhybridation des deux oligonucléotides est suivie d'une étape de ligation puis d'un traitement à l'ADN polymérase de T4. Le mélange réactionnel est ensuite ligué au vecteur pTG1757 (en remplacement du fragment correspondant) préalablement digéré par *Sph*I et *Nco*I et traité à l'ADN polymérase de T4. On vérifie par séquençage, dans les clones obtenus, le nombre d"'unités" de 50 pb de *pho4* ainsi que leur orientation. C'est ainsi que l'on génère pTG8607, pTG8608 et pTG8609 comprenant respectivement 1, 2 et 3 copies de région de régulation de 50 pb en orientation anti-sens par rapport à la TATA box du gène *adh.*

### EXEMPLE 2 : Construction de vecteurs d'expression du gène HV2 Lys 47 comportant en outre le gène activateur de Schizosaccharomyces pombe.

*A. clonage* du *gène activateur de Schizosaccharomyces pombe.*
   Le gène codant pour un produit activateur capable d'activer l'expression des gènes régulables par la thiamine est isolé par complémentation d'une souche mutante de *Schizosaccharomyces pombe* présentant une absence de dérépression de l'expression du gène *pho4* en l'absence de thiamine. On isole des fragments génomiques partiellement digérés par *Sau*3A d'une souche de *Schizosaccharomyces pombe* de type sauvage. Ils sont clones dans le site *Bam*HI du vecteur pUR19 (Barbet et al., 1992, Gene, *114*, 59-66) puis introduits dans la souche mutante *thi*1-23 *ura*4 D18 *pho*1-44 (Schweingruber et al., 1992, Genetics, 130, 445-449).
   Les transformants sont sélectionnés pour la prototrophie pour l'uracile et le 5-(2 hydroxyethyl)-4-methylthiazole (Zurlinden et Schweingruber, 1992, Gene, 117, 141-143). 14 transformants sont obtenus et leur ADN plasmidique est isolé selon le protocole décrit dans Moreno et al. (1991, Methods in Enzymology, 194, 795-823). L'ADN est ensuite amplifié dans *Escherichia coli* selon les méthodes standards (Maniatis et al., 1982, Molecular cloning : a laboratory manual, Cold Spring Harbor Laboratory, Cold Spring Harbor, NY). Quatre transformants présentent un insert de 4 kb qui, après transformation dans la souche mutante précédente, est capable de supprimer l'absence de dérépression. Ceci se traduit par la restauration d'une activité phosphatase acide dans un milieu dépourvu de thiamine, mesurée selon la technique décrite dans Schweingruber et al. (1986, J. Biol. Chem., 261, 15877-15882). Il est donc probable que l'insert de 4 kb comprenne un gène activateur de l'expression des gènes régulables par la thiamine *nmtl* et *pho4.*
   On détermine la séquence de la majorité de l'insert selon les techniques conventionnelles connues de l'homme de l'art. Les données permettent de mettre en évidence un cadre de lecture ouvert de 775 résidus dont la séquence est reportée dans l'identificateur de séquence NO :1 (SEQ ID NO :1).
*B. Usage combiné du gène activateur de Schizosaccharomyces pombe et d'une caissette d'expression de HV2 Lys 47 sous le contrôle d'une région promotrice issue du gène pho4.*
   Le fragment *Sph*I*-Eco*RI est isolé du plasmide pUR19 comprenant l'insert de 4 kb et traité par l'ADN polymérase T4 avant d'être introduit dans des vecteurs d'expression réplicatifs multicopies de *Schizosaccharomyces* comportant une cassette d'expression contrôlée par le promoteur *pho4* et des marqueurs de sélection appropriés. Un vecteur de ce type est représenté dans la Figure 2.
*C. Usage combiné du gène activateur de Schizosaccharomyces pombe et d'une cassette d'expression de HV2 Lys 47 sous le contrôle d'une région promotrice issue du gène nmtl.*
   On génère un fragment PCR de 188 pb à partir de l'ADN génomique isolé d'une souche de *Schizosaccharomyces pombe* par les techniques conventionnelles. On utilise les oligonucléotides OTG5217 et OTG3028 (décrits respectivement dans les SEQ ID NO : 21 et 22). On introduit le fragment *Sph*I et *Bam*HI ainsi généré entre les mêmes sites du vecteur pTG1757. On obtient le vecteur pTG5774.
   Le fragment d'ADN codant pour le produit activateur est inséré dans un vecteur réplicatif multicopie de *Schizosaccharomyces* comportant notamment une cassette d'expression contrôlée par le promoteur *nmtl.*

### EXEMPLE 3 : Production de HV2 Lys 47 en fonction du plasmide utilisé.

Les vecteurs d'expression des exemples 1 et 2 sont introduits dans une souche de *Schizosaccharomyces* et on évalue le niveau d'expression du fragment d'ADN codant de l'hirudine selon la méthodologie mentionnée ci-dessous.

Une souche de *Schizosaccharomyces pombe,* par exemple la souche D18 disponible à l'AFRC sous la référence 2036, est transformée par les vecteurs d'expression précédemmént cités. On utilise à titre de contrôle négatif la même souche transformée en parallèle par le vecteur pTG1758 (Figure 1) comportant une région promotrice tronquée réduite à la région promotrice minimale du gène *adh.* Le contrôle positif d'expression est constitué par pTG1757 permettant une expression constitutive de HV2 Lys 47, sous le contrôle de la région promotrice du gène *adh*.

Les souches transformées sont cultivées dans un milieu Kappeli supplémenté avec 2% de glucose et un mélange de vitamines comprenant notamment de la thiamine à une concentration finale de 0,002g/l (milieu thi⁺). Lorsque les cultures atteignent une DO (densité optique) à 600nm comprise entre 1 et 2, elles sont diluées à une DO d'environ 0,05 soit dans du milieu thi⁺ de composition telle qu'indiquée ci-dessus, soit dans un milieu Kappeli supplémenté avec 2% de glucose et un mélange de vitamines dépourvu en thiamine (milieu thi-). Des parties aliquotes de culture sont prélevées régulièrement durant la phase exponentielle de croissance ainsi qu'en fin de croissance (DO 600nm comprise entre 7 et 9).

On détermine pour chaque échantillon prélevé, la quantité d'hirudine sécrétée dans le milieu de culture. Bien qu'il soit possible de doser l'hirudine par toutes les techniques conventionnelles, on utilise la technique ELISA telle que décrite dans Koch et al., (1993, Analytical Biochemistry, *214,* 301-312) en mettant en oeuvre le couple d'anticorps monoclonaux MATG102 et MATG106 et, de l'hirudine titrée comme décrit dans la référence précitée. Par ailleurs, on peut également évaluer le niveau d'expression de l'hirudine par analyse Northern des ARNm isolés des cellules de *Schizosaccharomyces pombe.* On met en oeuvre une sonde capable de s'hybrider spécifiquement avec les séquences codant pour HV2 Lys 47, par exemple une sonde issue du fragment *Acc*I*-Sac*I de pTG1757. Mais d'autres sondes, telles que des oligonucléotides, peuvent être employées.

La tableau 2 résume les niveaux d'hirudine sécrétée par *Schizosaccharomyces pombe* transformée par chacun des plasmides indiqués et selon que le milieu de culture contient de la thiamine ou non (thi⁺ ou thi⁻) (voir également Figure 3).

**Tableau 2**

| Vecteur | Région 5' *pho4* | Orientation | TATA box | Niveau de HV2 Lys47 | |
|---|---|---|---|---|---|
| | | | | thi+ | thi- |
| pTG1757 | - | - | *adh* | +++ | +++ |
| pTG1758 | - | - | *adh* | - | - |
| pTG5701 | 40pb | sens | *adh* | - | ++ à +++ |
| pTG4734 | 50pb | sens | *adh* | - | +++ |
| pTG4735 | 99pb | sens | *adh* | - | +++ |
| pTG4736 | 147pb | sens | *adh* | - | +++ |
| pTG4737 | 199pb | sens | *adh* | - | +++ |
| pTG4738 | 388pb | sens | *adh* | - | ++ |
| pTG2735 | 642pb | sens | *adh* | - | +++ |
| pTG2734 | 642pb | sens | *pho4* | - | +++ |
| pTG4770 | 50pb | antisens | *adh* | - | +++ |

Des estimations de la quantité d'ARNm HV2 Lys 47 par Northern blot confirment ces résultats.

D'autre part, lorsqu'on analyse par Northern blot les ARNm présents dans les cellules de *Schizosaccharomyces pombe* transformées par pTG8607, pTG8608 ou pTG8609 et cultivées en présence de thiamine, on ne peut détecter de signal d'hybridation. Par contre lorsque les cellules sont cultivées dans un milieu dépourvu de thiamine, on observe une forte augmentation de la quantité d'ARNm HV2 Lys 47. L'intensité du signal d'hybridation corrèle le nombre de copies de la séquence *pho4* de 50 pb.

L'ensemble de ces résultats montre qu'en présence de thiamine, l'expression du gène d'intérêt est réprimée alors qu'en son absence on observe une forte induction, se traduisant' par la présence d'une quantité importante d'hirudine sécrétée dans le milieu de culture.

D'autre part, ces expériences ont permis d'identifier une région de régulation, que l'on peut qualifier de minimale, comprise dans la région promotrice du gène *pho4* de *Schizosaccharomyces pombe* et responsable de la régulation par la thiamine. Cette région de 40 à 50 pb est située en amont de la TATA box et est suffisante, à elle seule, pour conférer une telle régulation (répression en présence de thiamine et dérépression en absence de thiamine). Le fait que cette région fonctionne quelle que soit son orientation vis à vis de la TATA box est inattendu.

Enfin, la présence de plusieurs copies de cette région minimale en amont d'une TATA box a un effet synergique sur le niveau d'expression, effet qui semble corréler le nombre de copies de cette région.

### EXEMPLE 4 : Construction d'un vecteur d'expression de la protéine CFTR sous le contrôle d'une région promotrice hybride "pho4-adh".

Le vecteur pTG5960, issu du p poly III-I^{∗} (Lathe et al., Gene, 1987, *57,* 193-201) et modifié par l'insertion de la séquence codant pour la protéine CFTR humaine dont la séquence en acide aminé est divulguée dans Riordan et al (1989, *supra*), est digéré par les enzymes *Ava*I et *Xho*I*.* Le fragment *Ava*I-*Xho*I comprenant la séquence nucléotidique codant pour la protéine CFTR est traité par le grand fragment Klenow de l'ADN polymérase et inséré dans le vecteur d'expression de *Schizosaccharomyces pombe* pTG2735 digéré par *Sac*I et *Bam*HI et traité par l'ADN polymérase T4. On génère pTG5999.

Le fragment *Ava*I-*Xho*I-Klenow est introduit en parallèle dans le vecteur pTG1702 digéré par BamHI et traité au grand fragment Klenow de l'ADN polymérase pour donner pTG1753 (expression constitutive du gène CFTR sous le contrôle de la région promotrice du gène *adh* de *Schizosaccharomyces pombe).*

Après transformation de *Schizosaccharomyces pombe* D18 par pTG1753, aucun transformant ne produit de protéine CFTR. L'analyse par cartographie de restriction montre que les plasmides isolés des transformants sont réarrangés, par exemple au niveau de la séquence CFTR. Ces résultats indiquent que l'expression de la protéine CFTR est probablement toxique pour les cellules.

Après introduction de pTG5999 dans la souche *Schizosaccharomyces pombe* D18 selon le protocole indiqué précédemment, les transformants sont cultivés en présence ou en absence de thiamine. Des parties aliquotes de cellules sont prélevées au cours du temps et la production de la protéine CFTR déterminée par Western blot (Dalemans et al., 1992, Experimental Cell Research, 201, 235-240). Aucun signal n'apparaît dans les cultures placées dans un milieu thi⁺ (répression de l'expression due à la présence de thiamine), alors qu'on détecte une bande ayant la masse moléculaire attendue dans les cultures réalisées en absence de thiamine.

Ces résultats montrent l'utilité de la région promotrice issue du gène *pho4* pour la production de protéines toxiques.

### EXEMPLE 5 : Influence de la position de la région 50pb du gène pho4 responsable de la régulation par la thiamine par rapport à la TATA box.

Afin de déterminer si la proximité de cette région vis à vis de la TATA box est requise pour les fonctions d'activation et de répression, elle a été clonée à deux autres positions plus éloignées de la TATA box *adh.*

Le plasmide pTG6726 est obtenu par digestion de pTG4734 par l'enzyme de restriction *Nco*I*,* traitement à l'ADN polymérase du phage T4 et ligation. Dans ces conditions, l'unité 50pb *pho*4 se trouve séparé par 10pb de la TATA box du gène *adh.*

Le vecteur pTG5786 provient du clonage du fragment *Sph*I*-Nco*I isolé de pTG4734 et portant l'unité 50pb *pho*4 dans le site *Sal*I du pTG4766, après traitement par l'ADN polymérase T4. De ce fait, après ligation la distance séparant l'unité 50pb de la TATA box *adh* est de 40 pb. Le plasmide pTG4766 dérive du pTG1757 par introduction d'un site *Sal*I 30pb en amont de la TATA box *adh.* Cette modification n'a aucun effet sur l'activité du promoteur *adh* et la délétion des séquences situées en amont du site *Sal*I (tel que réalisé dans le pTG4766) inhibe l'activité promotrice (comportement comparable à pTG1758).

Enfin le vecteur pTG5787 a été construit d'une manière similaire à la différence que l'unité 50pb *pho*4 est clone dans l'orientation inverse. Dans ce cas l'éloignement par rapport à la TATA box est de 36pb.

Les plasmides pTG6729, pTG5786 et pTG5787 ont été introduits dans la levure D18. Après culture en présence ou en absence de thiamine, les transcrits synthétisés par ces différents transformants ont été analysés par Northern-blot. La quantité de transcrits présents dans la souche transformée avec le pTG6729, en absence de thiamine, est inférieure à celle obtenue avec la souche portant le plasmide pTG4734, mais, en présence de thiamine, la répression est conservée. En ce qui concerne les souches transformées avec les plasmides pTG5786 et pTG5787 l'efficacité de transcription est comparable quelles que soient les conditions de culture (plus ou moins thiamine), et elle est semblable à celle mesurée dans la souche portant le pTG4734 en absence de thiamine.

Ainsi, l'éloignement de 10pb de l'unité 50pb par rapport à la TATA box *adh* diminue sa capacité à activer la transcription, mais ne modifie pas la répression par la thiamine. Par contre, lorsque celle-ci se trouve éloignée de 40pb de la TATA box, ses propriétés d'activation restent intactes mais elle devient incapable de réprimer la transcription.

Il apparait que la proximité entre la région régulatrice de 50pb du gène *pho4* et la TATA box est nécessaire pour que la répression par la thiamine soit efficace.

## Revendications

1. Cassette d'expression comprenant un fragment d'ADN codant pour une protéine d'intérêt, placé sous le contrôle des éléments nécessaires à son expression, lesdits éléments comportant une région promotrice régulable par la thiamine et issue du gène *pho4* de *Schizosaccharomyces pombe.*

2. Cassette d'expression selon la revendication 1, caractérisée en ce que ladite région promotrice régulable par la thiamine comprend au moins une région de régulation conférant la régulation par la thiamine, ladite région de régulation étant placée en amont d'une région promotrice minimale.

3. Cassette d'expression selon la revendication 2, caractérisée en ce que ladite région de régulation est placée immédiatement en amont de la TATA box d'une région promotrice minimale.

4. Cassette d'expression selon la revendication 2 ou 3, caractérisée en ce que la région promotrice régulable par la thiamine comprend de 1 à 25, avantageusement de 1 à 7, et de préférence, de 11 à 4 régions de régulation.

5. Cassette d'expression selon l'une quelconque des revendications 2 à 4, caractérisée en ce que la ou les région(s) de régulation est (sont) dans une orientation sens ou antisens par rapport à ladite région promotrice minimale.

6. Cassette d'expression selon l'une quelconque des revendications 2 à 5, caractérisée en ce que la ou les région(s) de régulation est (sont) issue(s) du gène *pho4* de *Schizosaccharomyces pombe.*

7. Cassette d'expression selon la revendication 6, caractérisée en ce que la région de régulation comprend au moins 17 nucléotides d'une séquence telle que montrée dans l'identificateur de séquence NO: 2 et débutant au nucléotide en position +617 et se terminant au nucléotide en position +642.

8. Cassette d'expression selon la revendication 7, caractérisée en ce que la région de régulation est la séquence telle que montrée dans l'identificateur de séquence NO: 2
- débutant au nucléotide en position +603 et se terminant au nucléotide en position +642;
- débutant au nucléotide en position +593 et se terminant au nucléotide en position +642;
- débutant au nucléotide en position +544 et se terminant au nucléotide en position +642;
- débutant au nucléotide en position +496 et se terminant au nucléotide en position +642;
- débutant au nucléotide en position +444 et se terminant au nucléotide en position +642 ;
- débutant au nucléotide en position +255 et se terminant au nucléotide en position +642; ou - débutant au nucléotide en position +1 et se terminant au nucléotide en position +642.

9. Cassette d'expression selon l'une quelconque des revendications 2 à 8, caractérisée en ce que la région promotrice minimale est issue du gène *adh* de *Schizosaccharomyces pombe.*

10. Vecteur d'expression comprenant une cassette d'expression selon l'une quelconque des revendications 11 à 9.

11. Cellule hôte caractérisée en ce qu'elle comprend une cassette d'expression selon l'une quelconque des revendications 1 à 9 ou un vecteur d'expression selon la revendication 10.

12. Procédé de production d'une protéine d'intérêt par une cellule hôte selon la revendication 11, selon lequel :
- on cultive dans un milieu approprié en absence de thiamine ladite cellule hôte ; et
- on récupère ladite protéine d'intérêt.

13. Procédé selon la revendication 12, caractérisé en ce que la cellule hôte est une souche de *Schizosaccharomyces pombe.*

## Patentansprüche

1. Expressionskassette, die ein für ein interessierendes Protein kodierendes DNA-Fragment umfaßt, welches so angeordnet ist, daß es unter Kontrolle der für seine Expression notwendigen Elemente steht, wobei diese Elemente eine durch Thiamin regulierbare Promoter-Region umfassen, die aus dem Gen pho4 aus Schizosaccharomyces pombe stammt.

2. Expressionskassette nach Anspruch 1, dadurch charakterisiert, daß die durch Thiamin regulierbare Promoter-Region mindestens eine Regulationsregion umfaßt, die die Regulation durch Thiamin bewirkt, wobei die Regulationsregion stromaufwärts einer minimalen Promoter-Region angeordnet ist.

3. Expressionskassette nach Anspruch 2, dadurch charakterisiert, daß die Regulationsregion direkt stromaufwärts der TATA-Box einer minimalen Promoter-Region angeordnet ist.

4. Expressionskassette nach den Ansprüchen 2 oder 3, dadurch charakterisiert, daß die durch Thiamin regulierbare Promoter-Region 1 bis 25, vorteilhafterweise 1 bis 7 und bevorzugt 1 bis 4 Regulationsregionen umfaßt.

5. Expressionskassette nach einem der Ansprüche 2 bis 4, dadurch charakterisiert, daß die Regulationsregion(en) in einer Sense- oder Antisense-Orientierung, bezogen auf die minimale Regulationsregion, vorliegt (vorliegen).

6. Expressionskassette nach einem der Ansprüche 2 bis 5, dadurch charakterisiert, daß die Regulationsregion(en) aus dem Gen pho4 aus Schizosaccharomyces pombe stammt (stammen).

7. Expressionskassette nach Anspruch 6, dadurch charakterisiert, daß die Regulationsregion mindestens 17 Nucleotide einer im Sequenzprotokoll Nr. 2 dargestellten Sequenz umfassen und beim Nucleotid in Position +617 beginnt und beim Nucleotid in Position +642 endet.

8. Expressionskassette nach Anspruch 7, dadurch charakterisiert, daß die Regulationsregion die im Sequenzprotokoll No. 2 dargestellte Sequenz ist
- beginnend beim Nucleotid in Position +603 und endend beim Nucleotid in Position +642;
- beginnend beim Nucleotid in Position +593 und endend beim Nucleotid in Position +642;
- beginnend beim Nucleotid in Position +544 und endend beim Nucleotid in Position +642;
- beginnend beim Nucleotid in Position +496 und endend beim Nucleotid in Position +642;
- beginnend beim Nucleotid in Position +444 und endend beim Nucleotid in Position +642;
- beginnend beim Nucleotid in Position +255 und endend beim Nucleotid in Position +642;
- beginnend beim Nucleotid in Position +1 und endend beim Nucleotid in Position +642.

9. Expressionskassette nach einem der Ansprüche 2 bis 8, dadurch charakterisiert, daß die minimale Promoter-Region aus dem Gen adh aus Schizosaccharomyces pombe stammt.

10. Expressionsvektor, umfassend eine Expressionskassette nach einem der Ansprüche 1 bis 9.

11. Wirtszelle, dadurch charakterisiert, daß sie eine Expressionskassette nach einem der Ansprüche 1 bis 9 oder einen Expressionsvektor nach Anspruch 10 umfaßt.

12. Verfahren zur Herstellung eines interessierenden Proteins durch eine Wirtszelle nach Anspruch 11, nach dem
- man die Wirtszelle in einem geeigneten Medium in Abwesenheit von Thiamin kultiviert; und
- das interessierende Protein isoliert.

13. Verfahren nach Anspruch 12, dadurch charakterisiert, daß die Wirtszelle ein Stamm von Schizosaccharomyces pombe ist.

## Claims

1. Expression cassette comprising a DNA fragment coding for a protein of interest, placed under the control of the elements necessary for its expression, the said elements containing a thiamine-regulable promoter region originating from the *Schizosaccharomyces pombe pho4* gene.

2. Expression cassette according to Claim 1, characterized in that the said thiamine-regulable promoter region comprises at least one regulatory region conferring regulation by thiamine, the said regulatory region being placed upstream of a minimal promoter region.

3. Expression cassette according to Claim 2, characterized in that the said regulatory region is placed immediately upstream of the TATA box of a minimal promoter region.

4. Expression cassette according to Claim 2 or 3, characterized in that the thiamine-regulable promoter region comprises from 1 to 25, advantageously from 1 to 7 and preferably from 1 to 4 regulatory regions.

5. Expression cassette according to any one of Claims 2 to 4, characterized in that the regulatory region(s) is (are) in a sense or antisense orientation with respect to the said minimal promoter region.

6. Expression cassette according to any one of Claims 2 to 5, characterized in that the regulatory region(s) originate(s) from the *Schizosaccharomyces pombe pho4* gene.

7. Expression cassette according to Claim 6, characterized in that the regulatory region comprises at least 17 nucleotides of a sequence as shown in the sequence identifier NO: 2 and beginning at the nucleotide at position +617 and ending at the nucleotide at position +642.

8. Expression cassette according to Claim 7, characterized in that the regulatory region is the sequence as shown in the sequence identifier NO: 2
- beginning at the nucleotide at position +603 and ending at the nucleotide at position +642;
- beginning at the nucleotide at position +593 and ending at the nucleotide at position +642;
- beginning at the nucleotide at position +544 and ending at the nucleotide at position +642;
- beginning at the nucleotide at position +496 and ending at the nucleotide at position +642;
- beginning at the nucleotide at position +444 and ending at the nucleotide at position +642;
- beginning at the nucleotide at position +255 and ending at the nucleotide at position +642; or
- beginning at the nucleotide at position +1 and ending at the nucleotide at position +642.

9. Expression cassette according to any one of Claims 2 to 8, characterized in that the minimal promoter region originates from the *Schizosaccharomyces pombe adh* gene.

10. Expression vector comprising an expression cassette according to any one of Claims 1 to 9.

11. Host cell, characterized in that it comprises an expression cassette according to any one of Claims 1 to 9 or an expression vector according to Claim 10.

12. Method for the production of a protein of interest by a host cell according to Claim 11, according to which:
- the said host cell is cultured in a suitable medium in the absence of thiamine; and
- the said protein of interest is recovered.

13. Method according to Claim 12, characterized in that the host cell is a *Schizosaccharomyces pombe* strain.
